# EUROPEAN PATENT APPLICATION

(11) **EP 3 333 267 A1**
(43) Date of publication of application: **13.06.2018**
(21) Application number: 16203285.8
(22) Date of filing: 09.12.2016
(51) Int. Cl.: C12Q 1/68

(54) **METHOD AND KIT FOR PREDICTING THE RISK OF DEEP VEIN THROMBOSIS AND PULMONARY EMBOLISM**

(71) Applicant: Gene Predictis SA, 1015 Lausanne (CH)
(72) Inventor: TANACKOVIC ABBAS-TERKI, Goranka, 1015 Lausanne (CH); MICHAUD SCHUTZ, Joëlle, 1015 Lausanne (CH); PACHE, Thierry Daniel, 1015 Lausanne (CH)
(74) Representative: KATZAROV S.A.

(57) **Abstract**

The invention relates to the field of medicine, in particular to the field of blood clotting diseases prognosis. Specifically, the present invention relates to specific single nucleotide polymorphisms (SNPs) in the human genome, and their association with deep vein thrombosis (DVT) and related pathologies, such as pulmonary embolism (PE).

SEQUENCE LISTING

<110>  Gene Predictis SA

<120>  METHOD FOR PREDICTING THE RISK OF DEEP VEIN THROMBOSIS AND
       PULMONARY EMBOLISM

<130>  50603/EP

<160>  9

<170>  PatentIn version 3.5

<210>  1
<211>  51
<212>  DNA
<213>  Homo sapiens

<220>
<221>  misc_feature
<223>  "n" is C or T

<400>  1
gatggggaag aggagcattg aggacngtgt tcaagaggaa gcccgctgcc t                51

<210>  2
<211>  51
<212>  DNA
<213>  Homo sapiens

<220>
<221>  misc_feature
<223>  "n" is C or T

<400>  2
tggatggtat tgacttttac atcacngaag gtgtttccat agatggaaga c                51

<210>  3
<211>  51
<212>  DNA
<213>  Homo sapiens

<220>
<221>  misc_feature
<223>  "n" is A or G

<400>  3
tgtaagagca gatccctgga caggcnagga atacaggtat tttgtccttg a                51

<210>  4
<211>  51
<212>  DNA
<213>  Homo sapiens

<220>
<221>  misc_feature
<223>  "n" is A or G

<400>  4
gttcccaata aaagtgactc tcagynagcc tcaatgctcc cagtgctatt c          51

<210>  5
<211>  51
<212>  DNA
<213>  Homo sapiens

<220>
<221>  misc_feature
<223>  "n" is - or G

<400>  5
gcagtaggaa ggatgtcctc gtggtnaccc cttggctggc tcccattgtc t          51

<210>  6
<211>  51
<212>  DNA
<213>  Homo sapiens

<220>
<221>  misc_feature
<223>  "n" is - or C

<400>  6
ccaagaacca ccaggcggtc cggaanccgt gagcggctgc caggggctct g          51

<210>  7
<211>  51
<212>  DNA
<213>  Homo sapiens

<220>
<221>  misc_feature
<223>  "n" is C or G

<400>  7
gcgatgttaa ttactctcca gccccntcag aaggggctgg attgatggag g          51

<210>  8
<211>  51
<212>  DNA
<213>  Homo sapiens

<220>
<221>  misc_feature

<223>  "n" is C or T

<400>  8
gtgagggtga ggcttgtctc tctctngccc tctcatcctg gcacatgtgc g          51

<210>  9
<211>  51
<212>  DNA
<213>  Homo sapiens

<220>
<221>  misc_feature
<223>  "n" is A or G

<400>  9
agggatccaa tcgtcatcac tctgtntggg agctggtgat ataggaggca t          51

## Description

### TECHNICAL FIELD

The invention relates to the field of medicine, in particular to the field of blood clotting diseases prognosis. Specifically, the present invention relates to specific single nucleotide polymorphisms (SNPs) in the human genome, and their association with deep-vein thrombosis (DVT) and related pathologies, such as pulmonary embolism (PE).

### BACKGROUND OF THE INVENTION

Venous thrombosis is a serious medical condition that occurs when a blood clot (thrombus) forms in one or more veins of the body. One particular form of venous thrombosis is called deep vein thrombosis (DVT) when the blood clot occurs in deep veins. Such blood clots might travel through the bloodstream and lodge in lungs, where they can block blood flow, causing pulmonary embolism (PE).

Venous thrombosis is a complex multifactorial disease, several inherited or acquired conditions can increase coagulability of blood and thus the tendency to develop blood clots. The inherited conditions include mutations in the diverse well-known clotting anticoagulant or thrombolytic factors genes, such as the factor V Leiden gene, and the prothrombin factor II gene. Additional mutations can be present in genes coding for proteins C and S, although they increase risk of developing venous thrombosis significantly, they are rare and most of them are practically private. Other likely inherited causes include a possible increase in the expression of procoagulant factors such as factor VIII, von Willebrand factor, and factors IX and XI (Cushman M., (2005), Hematology Am Soc Hematol Educ Program: 452-457). In addition, non-O ABO blood groups, with exception of the A2 group, were demonstrated as increasing the risk of developing thrombosis; as well, as some additional genetic variants in the genes such as *FGG, GP6, KNG1*, *PROCR, SLC44A2*, *STXBP5* and *TSPAN15* were associated to an increased risk of venous thrombosis (Morange P-E., Suchon P. and Trégouët D-A., (2015), Thrombosis and Haemostasis 114 (5): 910-919). The examples of the acquired conditions that can cause DVT are surgery and trauma, prolonged immobilization, cancer, myeloproliferative disorders, and even pregnancy and post-partum (Seligsohn U. and Lubetsky A., (2001) New Eng J Med 344(16): 1222-1231). Thus, DVT might occur as the result of a genetic mutation alone or in concert with behavioral and environmental factors, such as prolonged immobilization, smoking and hormonal treatments (e.g. use of hormonal contraceptives or hormone replacement therapy for menopause).

Venous thrombosis occurs in 1-2 per 1'000 individuals per year. The incidence increases with age, from 1 in 100'000 in children to 1 in 10'000 individuals in the reproductive age and 1 in 1'000 individuals at the age 50 to 60 (Rosendaal F. R., (2016), Thromb J 14(Suppl 1): 117-121). As mentioned, numerous hereditary and acquired conditions were demonstrated in relation with an increased risk of developing DVT, nonetheless widely-accepted evidence of haemostatic abnormalities associated with thrombophilia includes the following parameters: antithrombin deficiency, protein S deficiency, protein C deficiency, factor V Leiden mutation, prothrombin *G20210A* mutation, non-O ABO blood groups, high levels of factor VIII dysfibrinogenaemia and hyperhomocysteinaemia (Franchini M., Martinelli I. and Mannucci P. M., (2016), Thrombosis and Haemostasis 115: 25-30). These parameters are also included in the standard thrombophilia testing, which thus includes only few genetic markers (the factor V Leiden mutation, the prothrombin *G20210A* mutation and eventually non-O blood groups.

Besides, over 100 million women worldwide use combined estroprogestative contraceptives (CC), due to their very high effectiveness in reducing the risk of unwanted pregnancy and their beneficial effect on diverse symptoms related to women's cycle. Nonetheless, these contraceptives also increase the risk of blood clotting substantially, which can ultimately lead to DVT and PE (Vinogradova Y., Coupland C. and Hippisley-Cox J., (2015) BMJ 350: h2135). Newer generations of the CC, the so-called 3rd and 4th generations CC, are usually better tolerated by women but importantly, they increase the risk of developing DVT even more than the older preparations of the so-called 2^{nd} generation.

The incidence of thrombosis among CC users is around 1‰. In France alone, where over 3 M women aged 15-49 use CC, the National Agency for the Safety of Drugs and Health Products reports every year over 2'500 cases of DVT, 850 cases of PE, and 20 cases of death linked to contraceptive pills. According to recent estimates, in Europe, 22'000 DVT cases related to CC occur each year. Thus, one of the major challenges for healthcare professionals is to identify women at risk of developing blood clotting disease related to CC such as DVT and PE, and advise them on alternative contraception methods.

As the standard of care nowadays, prescribing physicians use a medical questionnaire to assess the risk of thrombosis, mostly focusing on age, body mass index and smoking habits that are known risk factors for disease development, as well as on the personal and familial history of DVT or related diseases. If the familial or personal history of thrombosis is positive, physicians might use the first-level laboratory test for thrombophilia screening that includes analysis of only 2 genetic risk factors: the factor V-Leiden and the prothrombin mutation; eventually, some laboratories, also include genetic tests allowing to assess for the ABO blood groups. Though these genetic factors are well-established risk factors for thrombosis development, they explain less then one third of the inherited risk to develop thrombosis. Preciseley, factor V-Leiden is present among 20% of patients that develop thrombosis, whereas only 6% of patients carry the prothrombin mutation (Rosendaal F.R. and Reitsma P.H., (2009); Journal of Thrmobosis and Haemostasis 7(1): 301-304). Presence of the non-O ABO blood group, with exception of the A2 blood group, increases the risk of developing thrombosis but it was demonstrated as a particulary important risk factor when combined with factor V-Leiden mutation due to additive effect (Franchini M. and Mannucci P. M., (2014), Thrombosis and Haemostasis 112(6): 1103-1109).

Taking into account the currently registered number of thrombosis cases related to CC, as well as the small proportion of thrombosis patients that are carriers of the genetic variants included into the standard testing, this approach is a relatively low performing one, with suboptimal sensitivity and specificity. This observation is futher confirmed through diverse studies that demonstrate that these informations, notably familial history, are insufficient to reliably estimate risk of DVT (de Haan H.G. et al., (2012), Blood 120: 656-663; Suchon P, et al., (2015), Thrombosis and Haemostasis 114(6)).

For women in the period of menopause, hormone replacement therapy (HRT) aims to prevent discomfort caused by diminished circulating estrogen and progesterone hormones in woman's body, or in the case of the surgically or prematurely menopaused women, it aims at prolonging life and reducing the occurence of osteoporosis and dementia. It involves use of preparations that usually include estrogens and progesterone or progestins. As in the case of contraceptive treatments, these hormones and consequently the replacement treatments increase importantly the risk of developing DVT and PE. It is now well established that the presence of the factor V Leiden mutation and prothrombin mutation has a multiplicative effect on the overal risk of DVT related to homone replacement therapy (Douketis J.D. et al., (2011), Clin Appl Thromb Hemost. 17(6): E106-113; Botto N., et al., (2011), Climacteric. 14(1): 25-30).

Methods of genetic epidemiology estimate that heritability of DVT is around 50% (Morange P-E., Suchon P. and Trégouët D-A., (2015), Thrombosis and Haemostasis 114 (5): 910-919). Thus, considering the incidence of DVT and PE in human beings, there is a strong need to identify new genetic factors involved in the risk of developping blood clotting diseases.

### SUMMARY OF THE INVENTION

The present invention provides a prognostic method for identifying if a subject is at risk of developing a blood clotting disease, the method comprising determining in a sample from said subject the genotype of at least one single nucleotide polymorphism selected from the group consisting of rs1799853 (SEQ ID NO: 1) and rs4379368 (SEQ ID NO: 2).

The invention also provides a kit for use in said prognostic method, comprising
i) Detection reagents for detecting single nucleotide polymorphisms selected from the group consisting of rs1799853 (SEQ ID NO: 1) and rs4379368 (SEQ ID NO: 2); and optionally
ii) Instructions for use.

The invention further provides a computer program or a computer-readable media containing means for carrying out for identifying if a subject is at risk of developing a blood clotting disease, the method comprising determining in a sample from said subject the genotype of at least one single nucleotide polymorphism selected from the group consisting of rs1799853 (SEQ ID NO: 1) and rs4379368 (SEQ ID NO: 2).

### DETAILED DESCRIPTION OF THE INVENTION

The present invention relates to the identification of novel single nucleotide polymorphisms (SNPs) and unique combinations of such SNPs in a subject or patient, as well as combination of SNPs and clinical risk factors that are associated with a risk of developing blood clotting diseases.

In particular, the present invention provides a prognostic method for identifying if a subject is at risk of developing a blood clotting disease, the method comprising determining in a sample from said subject the genotype of at least one single nucleotide polymorphism (SNP) selected from the group consisting of rs1799853 (SEQ ID NO: 1) and rs4379368 (SEQ ID NO: 2).

As used in the specification and claims, the singular forms "a", "an" and "the" include plural references unless the context clearly dictates otherwise.

The term "comprise" is generally used in the sense of include, that is to say permitting the presence of one or more features or components.

As used herein the term "subject" or "patient" is used interchangeably herein to refer to both young and old human beings of both sexes. The term does not denote a particular age. For example, the subject is healthy or a subject is in need of a treatment or with a disease or disorder. Preferably, the subject is a human undergoing a change in hormone levels. More preferably, said subject is a woman undergoing a change in hormone levels. In another embodiment, the subject is a woman undergoing a change in particular female hormone levels, either induced by any treatment that involves hormone and/or by any particular naturally occurring change in a woman's life that modifies hormone levels. In particular, treatments include contraceptives, combined contraceptives, progestin-only contraceptives, hormone replacement therapy, assisted reproductive technology, and naturally occurring changes in a woman's life such as for example pregnancy and postpartum periods.

"Combined contraceptive" refers to any contraceptives that contain an estrogen combined with a progestin.

As used herein, the term "blood clotting disease" refers to diseases selected from the group comprising vein thrombosis, deep vein thrombosis (DVT), pulmonary embolism (PE) and arterial thrombosis.

As used herein, the term "at risk" when used with respect to developing a blood clotting disease refers to a subject which is more predisposed and likely to develop a blood clotting disease than a non "at risk" subject.

The present invention also provides a prognostic method for identifying if a subject undergoing a change in hormone levels is at risk of developing a blood clotting disease, the method comprising determining in a sample from said subject the genotype of rs1799853 (SEQ ID NO: 1) or rs4379368 (SEQ ID NO: 2).

Preferably, the present invention provides a prognostic method for identifying if a subject undergoing a change in hormone levels is at risk of developing a blood clotting disease, the method comprising determining in a sample from said subject the genotype of rs1799853 (SEQ ID NO: 1) and rs4379368 (SEQ ID NO: 2).

The invention also relates to a prognostic method for identifying if a subject is at risk of developing a blood clotting disease, the method comprising determining in a sample from said woman subject the genotype of at least one single nucleotide polymorphism (SNP) selected from the group consisting of rs1799853 (SEQ ID NO: 1) and/or rs4379368 (SEQ ID NO: 2), wherein said method further comprises determining the genotype of at least one single nucleotide polymorphism (SNP) selected from the group consisting of rs6025 (SEQ ID NO: 3), rs1799963 (SEQ ID NO: 4), rs8176719 (SEQ ID NO: 5), rs8176750 (SEQ ID NO: 6), rs9574 (SEQ ID NO: 7), rs2289252 (SEQ ID NO: 8), and rs710446 (SEQ ID NO: 9).

In a particular embodiment, the subject is a woman subject undergoing a change in hormone levels.

Thus, according to one embodiment, the invention relates to a prognostic method for identifying if a woman subject undergoing a change in hormone levels is at risk of developing a blood clotting disease, the method comprising determining in a sample from said woman subject the genotype of at least one single nucleotide polymorphism (SNP) selected from the group consisting of rs1799853 (SEQ ID NO: 1) and rs4379368 (SEQ ID NO: 2).

The present invention also provides a prognostic method for identifying if a woman subject undergoing a change in hormone levels is at risk of developing a blood clotting disease, the method comprising determining in a sample from said woman subject the genotype of rs1799853 (SEQ ID NO: 1) or rs4379368 (SEQ ID NO: 2).

Preferably, said method comprises determining in a sample from said woman subject the genotype of rs1799853 (SEQ ID NO: 1) and rs4379368 (SEQ ID NO: 2).

In another embodiment, the invention also relates to a prognostic method for identifying if a woman subject undergoing a change in hormone levels is at risk of developing a blood clotting disease, the method comprising determining in a sample from said woman subject the genotype of at least one single nucleotide polymorphism (SNP) selected from the group consisting of rs1799853 (SEQ ID NO: 1) and/or rs4379368 (SEQ ID NO: 2), wherein said method further comprises determining the genotype of at least one single nucleotide polymorphism (SNP) selected from the group consisting of rs6025 (SEQ ID NO: 3), rs1799963 (SEQ ID NO: 4), rs8176719 (SEQ ID NO: 5), rs8176750 (SEQ ID NO: 6), rs9574 (SEQ ID NO: 7), rs2289252 (SEQ ID NO: 8), and rs710446 (SEQ ID NO: 9).

Diverse forms of variations in a DNA sequence exist. Single nuleotide polymorphisms (SNPs) are the most common ones, representing more than 90% of all differences among individuals. Precisely, a SNP is a commonly occurring DNA variation (e.g. with frequency higher than 1%) in which two chromosomes differ in a given segment at a single position (a single base pair). Though SNPs are naturally and commonly occurring variations in human DNA, if they are part of the coding or regulatory DNA sequences, they might actually alter the expression of genes or stability of its transcripts and thus confer some advantages and risk to the carriers. It is now well established that these common genetic variants or their combination might be associated to some major traits, such as diseases. SNPs are well known to person skilled in the art and are notably described in the NCBI database dbSNP (www.ncbi.nlm.nih.gov/SNP/).

As used herein, the main SNPs that are concerned by the invention are described as follow (Table 1):

**Table 1**

| SEQ ID NO: | rs | Sequence | Gene |
|---|---|---|---|
| SEQ ID NO:1 | rs1799853 | GATGGGGAAGAGGAGCA TTGAGGAC[C/T]GTGTTCA AGAGGAAGCCCGCTGCCT | *CYP2C9* gene (NG_008385) |
| SEQ ID NO:2 | rs4379368 | TGGATGGTATTGACTTTTA CATCAC[C/T]GAAGGTGTT TCCATAGATGGAAGAC | *SUGCT* gene (NG_023422) |
| SEQ ID NO: 3 | rs6025 | TGTAAGAGCAGATCCCTG GACAGGC[A/G]AGGAATA CAGGTATTTTGTCCTTGA | *Factor V* gene (NG_011806) |
| SEQ ID NO: 4 | rs1799963 | GTTCCCAATAAAAGTGAC TCTCAGY[A/G]AGCCTCAA TGCTCCCAGTGCTATTC | *Factor II* gene (NG_008953) |
| SEQ ID NO:5 | rs8176719 | GCAGTAGGAAGGATGTCC TCGTGGT[- /G]ACCCCTTGGCTGGCTC CCATTGTCT | *ABO* gene (NG_006669) |
| SEQ ID NO:6 | rs8176750 | CCAAGAACCACCAGGCGG TCCGGAA[- /C]CCGTGAGCGGCTGCCA GGGGCTCTG | *ABO* gene (NG_006669) |
| SEQ ID NO:7 | rs9574 | GCGATGTTAATTACTCTCC AGCCCC[C/G]TCAGAAGG GGCTGGATTGATGGAGG | *PROCR* gene (NG_032899) |
| SEQ ID NO:8 | rs2289252 | GTGAGGGTGAGGCTTGTC TCTCTCT[C/T]GCCCTCTCA TCCTGGCACATGTGCG | *Factor 11* gene (NG_008051) |
| SEQ ID NO:9 | rs710446 | AGGGATCCAATCGTCATC ACTCTGT[A/G]TGGGAGCT GGTGATATAGGAGGCAT | *KNG1* gene (NG_016009) |

In the prognostic methods according to the invention, the genotype of the SNPs is determined by nucleic acid sequencing and/or by PCR analysis in a sample of the subject, preferably a woman subject undergoing a change in hormone levels.

The sample may be any biological sample, containing DNA and derived from the subject. This includes body fluids, tissues, cells, biopsies and so on. The preferred samples are saliva and blood.

The sample is collected according to the transfer method of choice and is treated to purify nucleic acids. These treatments include lysis, centrifugation and washing steps. The lysis includes mechanical, physical or chemical approaches. The purified nucleic acid is then used to genotype the above SNPs. The nucleic acid is added to a buffer, enzymes, specific primers and/or probes. This can be done in any suitable device such as tube, plate, well, glass etc. The genotype of the above SNPs may be detected by PCR, RFLP, allele-specific PCR, quantitative PCR, sequencing, microarray, hybridization, etc.

Sequencing can be performed using automatic sequencers using various techniques and following state of the art protocols. The sequencing reactions can be performed on complete genes or on specific regions covering the SNPs. For standard sequencing, the reaction requires a piece of DNA that initiates the amplification reaction, standard nucleotides and modified nucleotides that block the amplification reaction. Next generation sequencing can be performed by various techniques that massively amplified DNA regions.
Amplification can be performed using numerous techniques such as polymerase chain reaction (PCR), allele-specific PCR (ASA PCR), PCR followed by a restriction enzyme digestion (RFLP-PCR) and quantitative PCR. These techniques are based on the amplification of specific regions using small pieces of DNA that hybridize to the specific regions and initiate the amplification reaction. The reaction also requires necessary components to make the amplification work such as nucleotides and enzymes. In the case of ASA-PCR, a multiplex reaction or independent reactions are used by mixing 4 pieces of DNA that will initiate the amplification reaction. Among these 4 pieces of DNA, 2 are specific to the SNP; one will be specific to the effect allele and the other will be specific to the non-effect allele. The sizes of the amplified regions would be different to be able to distinguish between them on a regular agarose gel. In the case of RFLP-PCR, the amplified material is treated using a restriction enzyme to cut the amplified region according to the SNP allele. For example, enzyme A would cut the effect allele, while it would not cut the non-effect allele. The sizes of the digested amplified region are distinguished on a regular agarose gel. For quantitative PCR, labelled probes specific to each allele are added to the amplification reaction to distinguish between the two alleles.
Genotyping can also be performed using hybridization techniques on a solid support or in suspension. These techniques are based on the hybridization of material amplified with allele specific probes and primers onto a support. The amplified material is labelled to distinguish the different alleles.

The term "effect allele" according to the invention refers to the allele that confers the risk and/or the protective effect to develop a blood clotting disease to a subject, preferably a woman subject undergoing a change in hormone levels. The term "effect allele", thus refers to a SNP or allele that is associated with high relative risk of developing a blood clotting disease.

The term "non-effect allele" refers to the allele that does not confer a risk nor a protective effect to develop a blood clotting disease to a subject, preferably a woman subject undergoing a change in hormone levels.

In the context of the invention, prognostic methods are performed to determine the presence or absence of homozygous or heterozygous forms of at least one "effect allele". The genotyping data include three possibilities, which are homozygous for the "effect-allele", heterozygous, or homozygous for the "non-effect allele".

Table 2 gives the detailed genotypes for each SNP (A: Adenine, G: Guanine, C: Cytosine, T: Thymine, -: deletion).

**Table 2**

| | Genotype | | |
|---|---|---|---|
| SNP | Homozygous for the "effect allele" | Heterozygous | Homozygous for the "non-effect allele" |
| rs1799853 | (T;T) | (C;T) | (C;C) |
| rs4379368 | (T;T) | (C;T) | (C;C) |
| rs6025 | (A;A) | (A;G) | (G;G) |
| rs1799963 | (A;A) | (A;G) | (G;G) |
| rs8176719 | (G;G) | (-;G) | (-;-) |
| rs8176750 | (-;-) | (-;C) | (C;C) |
| rs9574 | (G;G) | (C;G) | (C;C) |
| rs2289252 | (T;T) | (C;T) | (C;C) |
| rs710446 | (G;G) | (A;G) | (A;A) |

In the above described prognostic methods, the presence of allele T in rs1799853 (SEQ ID NO: 1) and/or allele T in rs4379368 (SEQ ID NO: 2) in heterozygous or homozygous form indicates that said subject has an increased risk of developing a blood clotting disease.

In comparison, the presence of allele C in rs1799853 (SEQ ID NO: 1) and/or allele C in rs4379368 (SEQ ID NO: 2) in homozygous form indicates that said subject has no increased risk of developing a blood clotting disease.

Preferably, the presence of allele T in rs1799853 (SEQ ID NO: 1) and allele T in rs4379368 (SEQ ID NO: 2) in heterozygous or homozygous form indicates that said subject has an increased risk of developing a blood clotting disease.

More preferably, the presence of allele T in rs1799853 (SEQ ID NO: 1), and/or allele T in rs4379368 (SEQ ID NO: 2) and an allele in heterozygous or homozygous form in at least one SNP selected from the group comprising allele A in rs6025 (SEQ ID NO: 3), allele A in rs1799963 (SEQ ID NO: 4), allele G in rs8176719 (SEQ ID NO: 5), allele (-) in rs8176750 (SEQ ID NO: 6), allele G in rs9574 (SEQ ID NO: 7), allele T in rs2289252 (SEQ ID NO: 8), and allele G in rs710446 (SEQ ID NO: 9), indicates that said subject has an increased risk of developing a blood clotting disease.

As shown in the example, SNPs rs1799853 and rs4379368 provide statistically significant evidence of association with DVT and/or PE with a p-value of 5.10⁻⁴ and 3,6 .10⁻² respectively (Example 1, Table 5).

The present invention also relates to a prognostic method for identifying if a subject is at risk of developing a blood clotting disease, the method comprising the steps of:
a) determining in a sample from said subject the genotype of at least one single nucleotide polymorphism (SNP) selected from the group consisting of rs1799853 (SEQ ID NO: 1) and rs4379368 (SEQ ID NO: 2); and
b) determining the clinical risk factors of said subject.

The invention further provides a prognostic method for identifying if a subject is at risk of developing a blood clotting disease, the method comprising the steps of:
a) determining in a sample from said subject the genotype of at least one single nucleotide polymorphism (SNP) selected from the group consisting of rs1799853 (SEQ ID NO: 1) and rs4379368 (SEQ ID NO: 2);
b) determining in said sample the genotype of at least one single nucleotide polymorphisms (SNPs) selected from the group comprising rs6025 (SEQ ID NO: 3), rs1799963 (SEQ ID NO: 4), rs8176719 (SEQ ID NO: 5), rs8176750 (SEQ ID NO: 6), rs9574 (SEQ ID NO: 7), rs2289252 (SEQ ID NO: 8), and rs710446 (SEQ ID NO: 9); and
c) determining the clinical risk factors of said subject.

Preferably, the subject is a woman undergoing a change in hormone levels.

Clinical risk factors are for example selected from the group comprising the smoking status, the BMI, the age, the familial history of blood clotting diseases, the change in hormone levels, the personal history of blood clotting diseases, the alcohol status, a personal history of hypertension, of cholesterol, of diabetes, of autoimmune disease, of cancer and of other cardiovascular diseases, the history of contraception, the duration of the contraception, and the concomitant use of other drugs. For example, clinical risk factors may be determined through the request form sent by the physician or healthcare provider.

Preferably, clinical risk factors are selected from the group comprising the smoking status, the BMI, the age, the familial history of blood clotting diseases and/or the change in hormone levels.

The invention also relates to prognostic methods as described above, wherein said woman subject undergoing a change in hormone levels is having a contraceptive, a combined contraceptive, a hormonal replacement therapy, a progestin-only contraceptive, is pregnant, is having assisted reproductive technology, or is in postpartum period.

The type of hormone present in the subject's treatment includes the progestins levonorgestrel, norgestimate, gestoden, desogestrel, drospirenone, dienogest, cyproterone acetate, in particular but not exclusively and also the estrogens estradiol, ethinylestradiol, estradiol acetate, estradiol cypionate, estradiol valerate, estradiol enanthate, estradiol benzoate, estradiol hemihydrate, in particular but not exclusively. It also includes progestin-only pills, intrauterine devices and hormone replacement therapy according to the route of administration such as oral or transdermal patch and other therapy such as Livial (Tibolone).

In the prognostic methods of the invention, blood clotting diseases are selected from the group comprising deep vein thrombosis, pulmonary embolism, vein thrombosis and arterial thrombosis.

Preferably, the present invention provides prognostic methods for identifying if a woman subject undergoing a change in hormone levels is at risk of developing a deep vein thrombosis and /or a pulmonary embolism.

The invention further provides a kit for use in the prognostic methods described above, comprising:
i) Detection reagents for detecting SNP selected from the group consisting of rs1799853 (SEQ ID NO: 1) and/or rs4379368 (SEQ ID NO: 2); and optionally
ii) Instructions for use.

In a separate embodiment, a kit for use in the prognostic methods described above comprises:
i) Detection reagents for detecting SNP selected from the group consisting of rs6025 (SEQ ID NO: 3), rs1799963 (SEQ ID NO: 4), rs8176719 (SEQ ID NO: 5), rs8176750 (SEQ ID NO: 6), rs9574 (SEQ ID NO: 7), rs2289252 (SEQ ID NO: 8), rs710446 (SEQ ID NO: 9), rs4379368 (SEQ ID NO: 2) and rs1799853 (SEQ ID NO: 1); and optionally
ii) Instructions for use.

In particular, the kit contains necessary components to genotype the SNPs selected from the group comprising rs6025, rs1799963, rs8176719, rs8176750, rs9574, rs2289252, rs710446, rs4379368 and rs1799853. These components include primers and/or probes specific to each SNP. The primers are necessary to initiate the amplification of each specific region corresponding to each SNP. The probes hybridize specifically to each allele of each SNP and allow the detection of each allele. The probes for each allele can be labelled with different fluorophores to allow distinct detection. The kit can also contain solid support to hybridize amplified material and allow the detection of each allele on the solid support after labelling reaction. The kit can also contain necessary reagents to sequence the regions around the 9 SNPs listed above or the full corresponding genes. These reagents include specific primers for each primer that would allow initiating the amplification reaction and modified nucleotides.

Preferably, the SNP detection reagent is an isolated or synthetic DNA oligonucleotide probe or primer, or a RNA oligonucleotide or primer or a PNA oligomer or a combination thereof, that hybridizes to a fragment of a target nucleic acid molecule containing one of the SNPs specified in any one of SEQ ID Nos. 1 to 9, or a complement thereof.

In particular, said SNP detection reagent can differentiate between nucleic acids having a particular nucleotide at a target SNP position.

In the present invention, the SNP detection reagent hybridizes under stringent conditions to at least 8, 10, 12, 16, 18, 20, 22, 25, 30, 40, 50, 55, 60, 65, 70, 80, 90, 100, 120 ormore consecutive nucleotides in a target nucleic acid molecule comprising at least one of the SNPs specified in any one of SEQ ID Nos. 1 to 9, or a complement thereof.

Preferably, according to the invention, at least one SNP detection reagent in the kit is an oligonucleotide or primer having a length of at least 8 nucleotides, preferably a length of at least 10, 12, 16, 17, 18, 19, 20, 21, 22, 23, 24 or 25 nucleotides.

The invention also relates to a kit for use, wherein the SNP detection reagent is a compound that is labelled.

The present invention further relates to a computer program or a computer-readable media containing means for carrying out a prognostic method for identifying if a subject is at risk of developing a blood clotting disease, the method comprises determining in a sample from said subject the genotype of at least one single nucleotide polymorphism (SNP) selected from the group consisting of rs1799853 (SEQ ID NO: 1) and rs4379368 (SEQ ID NO: 2).

Preferably, said method further comprises the step of determining the genotype of at least one single nucleotide polymorphism (SNP) selected from the group consisting of rs6025 (SEQ ID NO: 3), rs1799963 (SEQ ID NO: 4), rs8176719 (SEQ ID NO: 5), rs8176750 (SEQ ID NO: 6), rs9574 (SEQ ID NO: 7), rs2289252 (SEQ ID NO: 8), and rs710446 (SEQ ID NO: 9).
The above-described methods may further comprises the step of determining the clinical risk factors of said subject.

In a separate embodiment, the present invention further relates to a computer program or a computer-readable media containing means for carrying out a prognostic method for identifying if a woman undergoing a change in hormone levels is at risk of developing a blood clotting disease, the method comprises determining in a sample from said woman the genotype of at least one single nucleotide polymorphism (SNP) selected from the group consisting of rs1799853 (SEQ ID NO: 1) and rs4379368 (SEQ ID NO: 2).

Preferably, said method further comprises the step of determining the genotype of at least one single nucleotide polymorphism (SNP) selected from the group consisting of rs6025 (SEQ ID NO: 3), rs1799963 (SEQ ID NO: 4), rs8176719 (SEQ ID NO: 5), rs8176750 (SEQ ID NO: 6), rs9574 (SEQ ID NO: 7), rs2289252 (SEQ ID NO: 8), and rs710446 (SEQ ID NO: 9).

The above-described methods may further comprises the step of determining the clinical risk factors of said woman undergoing a change in hormone levels.

Further SNPs can be determined based on the SNPs described in the present invention by statistical correlation. "Linkage disequilibrium" (LD) refers to the statistical correlation between two neighboring SNPs. LD is generally quantified with either Lewontin's parameter of association (D', Lewontin, R. C. (1964). Genetics. 49 (1): 49-67) or with the r² parameter based on Pearson correlation coefficient (Karl Pearson (1895) Proceedings of the Royal Society of London, 58: 240-242). When the LD value is equal to 1, the two SNPs are in complete disequilibrium. In contrast, two SNPs with a LD value equal to 0 are in complete linkage equilibrium. Linkage disequilibrium is calculated following the application of the expectation maximization algorithm (EM) for the estimation of haplotype frequencies.

Thus, in the present invention, SNPs considered in LD with rs1799853 (SEQ ID NO: 1), rs4379368 (SEQ ID NO: 2), rs6025 (SEQ ID NO: 3), rs1799963 (SEQ ID NO: 4), rs8176719 (SEQ ID NO: 5), rs8176750 (SEQ ID NO: 6), rs9574 (SEQ ID NO: 7), rs2289252 (SEQ ID NO: 8), or rs710446 (SEQ ID NO: 9) are SNPs with a r² greater than 0.5.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this specification, each of which is incorporated herein by reference in its entirety.

The foregoing description will be more fully understood with reference to the following Examples. Such Examples, are, however, exemplary of methods of practising the present invention and are not intended to limit the scope of the invention.

### EXAMPLE

### Example 1: Determination of the risk for women under hormonal contraceptives

### Characteristics of the studied population

Among 1622 women taking an oral contraceptive pill, 794 have developed a thrombotic event, either a deep vein thrombosis (DVT) or a pulmonary embolism (PE). Distribution of age, BMI and smoking status are presented in both populations (Table 3). Age distribution is similar in both groups, BMI and Smoking status are slightly higher in cases.

**Table 3**

| Clinical characteristics | Subject (n=794) | Controls (n= 828) |
|---|---|---|
| Age (years) | 32.3 | 31.5 |
| Body Mass Index (kg/m2) | 24 | 22.3 |
| Smoking status | 260 | 167 |

### Genotyping

50 genetic polymorphisms were identified for all 1622 women in the study using Illumina's Veracode-BeadXpress technology. The selection of these SNPs to be tested was made according to the meta-analyses of the existing literature and the laboratory studies that were performed. The quality of the genotyping data was assessed by GenomeStudio and by visual inspection. Genotyping data were validated by sequencing or RFLP-PCR.

### Statistical analyses

The cohort was randomly divided into a training set and a test set of equal size. The training set was used to perform a stepwise selection (AIC, Akaike information criterion) and a logistic regression to generate coefficients. The fitted model was then applied to the test dataset to compute predictions. This process was repeated 10'000 times and the medians of the coefficients were used. The variables not selected in the run were assigned a null coefficient.

The Odd-Ratio (OR) quantifies the association between two parameters in a given population (Cornfield J., (1951). Journal of the National Cancer Institute. 11: 1269-1275). These values are obtained from mathematical models such as the logistic regression used herein. A logistic regression is a mathematical model that measures the relationship between variables by predicting the probability of a given outcome (Walker, S.H.; Duncan, D.B. (1967). Biometrika. 54: 167-178). The OR and p-values are the output of the logistic regression and correspond to the strength and the significance of the measured relationship respectively.

### Results

This approach confirmed the association between DVT and/or PE with a number of SNPs already described in the literature (table 4). The table includes the Odd-Ratio (OR) observed in the literature, the literature references and the p-value obtained from the present described study. The Odd-Ratio indicates the association between a SNP and the development of thrombosis.

**Table 4**

| SNP | Gene | Change m/M | OR¹ | References² | p-value³ |
|---|---|---|---|---|---|
| rs6025 | *Factor V* gene (NG_011806) | G>A | 4.3 | De Haan, 2012 | 8.3x10⁻¹⁴ |
| rs1799963 | *Factor II* gene (NG_008953) | G>A | 3 | De Haan, 2012 | 3.7x10⁻⁸ |
| rs8176719 | *ABO* gene (NG_006669) | ->G | 1.74 | De Haan, 2012 | 6.0x10⁻⁷ |
| rs8176750 | *ABO* gene (NG_006669) | C>- | 1.89-1.91 | Tregouet, 2009 | 2.5x10⁻³ |
| rs9574 | *PROCR* gene (NG_032899) | C>G | 1.18⁺ | De Haan, 2012 | 9x10⁻⁴ |
| rs2289252 | *Factor 11* gene (NG_008051) | C>T | 1.36 | De Haan, 2012 | 2.9x10⁻⁴ |
| rs710446 | *KNG1* gene (NG_016009) | A>G | 1.196 | Morange, 2011 | 3.8x10⁻² |

| | | | | | |
|---|---|---|---|---|---|
| ¹ Odd-Ratio, for the association between the SNP and the development of thrombosis, obtained from the literature. Most of these OR are based on MARTHA cohort. ⁺ This OR corresponds to the SNP rs867186. Bothrs9574 andrs867186 are part of the haplotype A2 and A3 that are associated with the risk of thrombosis. ²References for the related OR ³ p-values obtained from the cohort of 1622 women and a logistic regression | | | | | |

Significant p-values were also obtained for two additional SNPs that were not previously associated with the development of thromboembolic events. These two SNPs are described in table 5:

**Table 5**

| SNP | Gene | Change (m/M) | p-value³ | OR⁴ |
|---|---|---|---|---|
| rs1799853 | *CYP2C9* gene (NG 008385) | C>T | 5.0x10⁻⁴ | 1.55 |
| rs4379368 | *SUGCT* gene (NG 023422) | C>T | 3.6x10⁻² | 1.35 |

| | | | | |
|---|---|---|---|---|
| ³ p-values obtained from the cohort of 1622 women and a logistic regression ⁴ Odd-Ratio obtained from the cohort of 1622 women and a logistic regression | | | | |

rs1799853 (SEQ ID NO: 1) is present in the gene coding for the cytochrome CYP2C9. This SNP is also known as the allele *2 in official nomenclature for the CYP450 enzymes. This variant is associated with a strong diminution of the enzyme's activity (Thijssen H.H., (2005), Clin Pharmacol Ther., 74, 61-68).

Ethinylestradiol that is the estrogen present in most of CC is metabolised via CYP450 pathways. Several of the cytochromes are involved including CYP2C9 (Lee AJ., (2003), Endocrinology, 144(8):3382-98).

rs4379368 (SEQ ID NO: 2) is present in the gene sequence coding for the succinyl-CoA:glutarate-CoA transferase. This SNP was initially found to be associated with migraine (Anttila V, (2013) Nat. Gen., 45(8):912-7).

ROC stands for Receiver Operating Characteristic and measures how well the different models discriminate the women at risk to the women without risk. The true positive rate (TPR) is plotted against the false positive rate (FPR) at various threshold settings (Fawcett T., (2006), Pattern Recognition Letters., 27: 861-874).

AUC stands for Area Under the Curve and is equal to the probability that a positive test ranks higher than a negative test in order to discriminate the women at risk to the women without risk. The AUC (Area Under the Curve) ranges from 0.5 (50% - no predictive value) to 1 (100% - perfect discrimination; Fawcett T., (2006), Pattern Recognition Letters., 27: 861-874)..

The AUC, obtained following the logistic regression described herein and corresponding to the different combinations of SNPs are shown in table 6.

**Table 6**

| rs and SEQ ID NO: | AUC |
|---|---|
| rs6025 (SEQ ID NO: 3) | 0.60 |
| rs1799963 (SEQ ID NO: 4) | |
| rs6025 (SEQ ID NO: 3) | 0.63 |
| rs1799963 (SEQ ID NO: 4) | |
| rs1799853 (SEQ ID NO: 1) | |
| rs6025 (SEQ ID NO: 3) | 0.62 |
| rs1799963 (SEQ ID NO: 4) | |
| rs4379368 (SEQ ID NO: 2) | |
| rs6025 (SEQ ID NO: 3) | 0.64 |
| rs1799963 (SEQ ID NO: 4) | |
| rs1799853 (SEQ ID NO: 1) | |
| rs4379368 (SEQ ID NO: 2) | |
| rs6025 (SEQ ID NO: 3) | 0.70 |
| rs1799963 (SEQ ID NO: 4) | |
| rs8176719 (SEQ ID NO: 5) | |
| rs8176750 (SEQ ID NO: 6) | |
| rs9574 (SEQ ID NO: 7) | |
| rs2289252 (SEQ ID NO: 8) | |
| rs710446 (SEQ ID NO: 9) | |
| rs4379368 (SEQ ID NO: 2) | |
| rs1799853 (SEQ ID NO: 1) | |

The values of the AUC demonstrate that rs4379368 (SEQ ID NO: 2), and/or rs1799853 (SEQ ID NO: 1) can be used in a prognostic method for identifying if a woman subject undergoing a change in hormone levels is at risk of developing a blood clotting disease.

In addition to rs4379368 (SEQ ID NO: 2), and/or rs1799853 (SEQ ID NO: 1), testing rs6025 (SEQ ID NO: 3), rs1799963 (SEQ ID NO: 4), rs8176719 (SEQ ID NO: 5), rs8176750 (SEQ ID NO: 6), rs9574 (SEQ ID NO: 7), rs2289252 (SEQ ID NO: 8), rs710446 (SEQ ID NO: 9) is advantageous in a prognostic method for identifying if a subject and particularly a woman subject undergoing a change in hormone levels is at risk of developing a blood clotting disease.

In the context of the invention, a report will indicate whether the subject is heterozygous or homozygous for the effect allele, or homozygous for the non-effect allele. The report will include a probability score to develop a blood clotting disease.

For calculating the score, the risk factor determined for a single nucleotide polymorphism of the invention may be pondered by a coefficient depending on what is the contribution of said single nucleotide polymorphism in the determination of the risk in comparison with the other one single nucleotide polymorphism. Typically, the method for calculating the score is based on statistical studies performed on various cohorts of patients. The score may also include other various patient parameters (such as clinical parameters). The weight given to each parameter is based on its contribution relative to the other parameters in explaining the inter-individual variability of developing a blood clotting disease.

This probability is derived from a model that includes the risk factor determined for the rs6025 (SEQ ID NO: 3); rs1799963 (SEQ ID NO: 4); rs8176719 (SEQ ID NO: 5); rs8176750 (SEQ ID NO: 6); rs9574 (SEQ ID NO: 7); rs2289252 (SEQ ID NO: 8); rs710446 (SEQ ID NO: 9); rs4379368 (SEQ ID NO: 2); and/or rs1 799853 (SEQ ID NO: 9) or any SNP in linkage disequilibrium with those SNPs cited above.

The probability score may also include clinical parameters (e.g. smoking status, the BMI, the age, the familial history of blood clotting diseases and/or the change in hormone levels) and the type of hormone used by the subject. The probability score may also be generated from a computer program for establishing such a score.

## Claims

1. A prognostic method for identifying if a subject is at risk of developing a blood clotting disease, the method comprising determining in a sample from said subject the genotype of at least one single nucleotide polymorphism selected from the group consisting of rs1799853 (SEQ ID NO: 1) and rs4379368 (SEQ ID NO: 2).

2. The prognostic method according to claim 1, comprising determining in a sample from said subject the genotype of rs1799853 (SEQ ID NO: 1) and rs4379368 (SEQ ID NO: 2).

3. The prognostic method according to any of claim 1 or 2, wherein the presence of allele T in rs1799853 (SEQ ID NO: 1) and/or allele T in rs4379368 (SEQ ID NO: 2) in heterozygous or homozygous form indicates that said subj ect has an increased risk of developing a blood clotting disease.

4. The prognostic method according to any of the claims 1 to 3, wherein said method further comprises determining the genotype of at least one single nucleotide polymorphism selected from the group consisting of rs6025 (SEQ ID NO: 3), rs1799963 (SEQ ID NO: 4), rs8176719 (SEQ ID NO: 5), rs8176750 (SEQ ID NO: 6), rs9574 (SEQ ID NO: 7), rs2289252 (SEQ ID NO: 8), and rs710446 (SEQ ID NO: 9).

5. The prognostic method according to any of the preceding claims, wherein the subject is a woman undergoing a change in hormone levels.

6. The prognostic method according to claim 5, wherein said woman subject undergoing a change in hormone levels is having a contraceptive, a combined contraceptive, a hormonal replacement therapy, a progestin-only contraceptive, is pregnant, is having assisted reproductive technology, or is in postpartum period.

7. The prognostic method according to any of the preceding claims, further comprising determining the clinical risk factors of said subject, said clinical risk factors are selected from the group comprising the smoking status, the BMI, the age, the familial history of blood clotting diseases and the change in hormone levels.

8. The prognostic method according to any of the preceding claims, wherein said blood clotting diseases is selected from the group comprising deep vein thrombosis, pulmonary embolism, vein thrombosis and arterial thrombosis.

9. The prognostic method according to any of the preceding claims, wherein the presence or absence of at least one single nucleotide polymorphism is determined by nucleic acid sequencing and/or by PCR analysis.

10. A kit for use in the prognostic method according to any of the preceding claims, comprising:
i) Detection reagents for detecting single nucleotide polymorphisms selected from the group consisting of rs1799853 (SEQ ID NO: 1) and rs4379368 (SEQ ID NO: 2); and optionally
ii) Instructions for use.

11. The kit for use in the prognostic method according to claim 10 further comprising detection reagents for detecting single nucleotide polymorphisms selected from the group consisting of rs6025 (SEQ ID NO: 3), rs1799963 (SEQ ID NO: 4), rs8176719 (SEQ ID NO: 5), rs8176750 (SEQ ID NO: 6), rs9574 (SEQ ID NO: 7), rs2289252 (SEQ ID NO: 8), and rs710446 (SEQ ID NO: 9).

12. The kit for use according to claims 10-11, wherein the single nucleotide polymorphism detection reagents are an isolated or synthetic DNA oligonucleotide probe or primer, or a RNA oligonucleotide or primer or a PNA oligomer or a combination thereof, that hybridizes to a fragment of a target nucleic acid molecule containing one of the single nucleotide polymorphisms specified in any one of SEQ ID Nos. 1 to 9, or a complement thereof.

13. A computer program or a computer-readable media containing means for carrying out the prognostic method according to any one of claims 1 to 9.
